# EUROPEAN PATENT SPECIFICATION

(11) **EP 2 278 956 B1**
(45) Date of publication and mention of the grant of the patent: **21.09.2011**
(21) Application number: 09723439.7
(22) Date of filing: 17.03.2009
(51) Int. Cl.: A61K 9/107, A61K 31/52, A61K 47/10, A61K 9/00, A61P 31/22

(54) **ANTIVIRAL FORMULATION**
ANTIVIRALE FORMULIERUNG
FORMULATION ANTIVIRALE

(30) Priority: 17.03.2008 EP 08152862; 18.09.2008 JP 2008238844
(43) Date of publication of application: 02.02.2011
(73) Proprietor: Medivir AB, 141 22 Huddinge (SE)
(72) Inventor: LARSSON, Torbjorn, S-141 22 Huddinge (SE)
(74) Representative: Goodall, Scott
(86) International application number: PCT/EP2009/053125
(87) International publication number: WO 2009/115510

(56) References cited:
- WO-A-00/29027
- WO-A-95/03805
- US-A- 4 963 555
- FIDDIAN A P ET AL: "Successful treatment of herpes labialis with topical acyclovir." BRITISH MEDICAL JOURNAL (CLINICAL RESEARCH ED.) 28 MAY 1983, vol. 286, no. 6379, 28 May 1983 (1983-05-28), pages 1699-1701, XP009100156 ISSN: 0267-0623

## Description

### Technical Field

This invention relates to topical antiviral formulations suitable for orofacial or genital application and comprising an acyclic guanosine antiviral agent. The invention further relates to the treatment or prophylaxis of herpesvirus diseases using such formulations and to their preparation.

### Background Art

Guanosine nucleoside analogues such as acyclovir, penciclovir or omacivlovir are efficacious against various herpesviruses, such as herpes simplex type 1 or 2 in cell culture experiments. However, these agents are notoriously difficult to formulate in conventional topical vehicles.

Topical acyclovir was initially approved for marketing as an ointment, although the evidence for efficacy was sparse and early publications showed varying results (Worrall 1991 Can. Fam. Physician 37:92-98).

European patent application no. EP 44543 relates to oil-in-water formulations of the acyclic nucleoside antiviral agent acyclovir and describes that effective topical penetration necessitates that the carrier comprises at least 30 weight percent, preferably at least 40 weight percent propylene glycol. This formulation, denoted the MAC formulation, forms the basis of the most widely marketed topical acyclovir preparation.

Phase 3 clinical trials using a robust and modern protocol for acyclovir in the MAC formulation are reported in Spruance et al 2002 Antimicrob. Agents Chemother. 46(7) 2238-2243. The trials were large-scale, randomised and placebo controlled. In the first study of 686 treated patients, the episode duration was reduced by 0.5 days (10%, P = 0.007) and the lesion pain duration was reduced by 0.3 days (9%, P = 0.017). In the second study (699 treated patients), the episode duration was reduced by 0.6 days (12%, P= 0.006) and lesion pain duration by 0.4 days (11%, P= 0.014).

Spruance further discusses almost identical results obtained for the cyclic guanosine analogue penciclovir (reduction in healing time 0.7 days, reduction of time to loss of pain 0.6 days).

Clearly these reductions are modest. Crucially, however, Prof Spruance reports "ACV cream did not prevent the development of classical lesions". In other words, although acyclovir in the cream vehicle had some effect in making cold sore lesions heal more quickly, and less painfully, it was not able to prevent cold sore lesions from arising, even when applied at the prodromal stage, 5 times daily for 4 days. The phenomenon of treatment-induced prevention of lesions is also referred to as "aborted lesions" and represents the holy grail of herpes simplex treatment.

Trottet et al 2005 Int. J. Pharm. 304:63-71 describes an analysis of acyclovir delivery from formulations with varying propylene glycol content. The authors found that the 40% propylene glycol formulation delivered 10 fold more acyclovir than the nearly identical formulation containing only 15% propylene glycol.

Various attempts have been made to improve the performance of topical acyclovir formulations, for example WO94/15614 (alkali oleate vehicle), WO90/03163 (choline ester vehicle), WO94/05258 (glycerol formate vehicle), WO96/35412 & WO98/02184 (phospholipid vehicles) WO97/34607 (diethylene glycol monoethyl ether vehicle). However, as far as we are aware, no improvement in aborted lesions has been achieved.

More recently attempts have been made to iontophoretically transport topical acyclovir into the dermal tissues with the use of a hand-held electric device.

International patent application no. WO91/11187 relates to oil-in-water or aqueous topical formulations of the guanosine antiviral penciclovir. These formulations must comprise at least 30 weight percent, preferably at least 35 weight percent, propylene glycol. European patent application no. EP 416 739 relates to topical formulations of penciclovir comprising at least 30 weight percent propylene glycol and a decyl methyl sulfoxide emulsifier. International patent application no. WO93/00905 relates to topical formulations of penciclovir comprising at least 30 weight percent, preferably at least 35 weight percent, propylene glycol and a cetomacrogol 1000 emulsifier.

WO95/03805 (Wellcome Foundation) describes various approaches to co-formulate acyclovir with the metabolically unstable ribonucleotide reductase inhibitor 2-acetylpyridine-5-[(2-chloroanilino)thiocarbonyl] thiocarbonohydrazone (also known as BW348U87 or TCH). As reported in Safrin et al 1993 Antimicro. Ag. Chemother. 975-979, phase II clinical trials with this combination had produced disappointing results, probably due to inadequate delivery of study drug to the affected area by the topical route of administration. However these co-formulation activities do not seem to have been successful and GSK, the successor to Wellcome, confirmed in a company statement on 16 November 2000 that the development had been terminated.

An alternative approach to enhancing the efficacy of topical guanosine antivirals is described in Evans et al 2002 Antimicrob. Agents Chemother. 46(6) 1870-1874. This reference describes a phase II clinical trial in a UV induced protocol, employing an antiviral/immunomodulatory combination of 5% acyclovir and 1% hydrocortisone. Healing time was reduced by 1.1 days (P = 0.04) and there was a trend toward a reduction in maximum lesion size (P = 0.07). Evans et al also draws parallels to other clinical trials in which high dose famciclovir (the oral prodrug of penciclovir) is co-administered with fluocinonide, a topical glucocorticoid immunomodulator. In these trials, patients receiving both the antiviral and glucocorticoid experienced aborted lesions 41 % of the time, whereas the frequency of aborted lesions dropped to just 8% in those patients receiving only oral famciclovir.

Co-formulation of (typically hydrophilic) guanosine antivirals such as acyclovir with (typically lipophilic) glucocorticoids such as hydrocortisone is not straightforward in view of the very different physicochemical properties of the actives. Short shelf life, unstable emulsions and crystal growth are frequent difficulties when conventional acyclovir formulations are used in combination with a glucocorticoid. To resolve these difficulties, WO00/29027 discloses a co-formulated combination of hydrocortisone and a guanosine antiviral in an oil-in water emulsion comprising a relatively low proportion of propylene glycol and isopropyl myristate.

### Brief Description of the Invention

We have surprisingly discovered that omission of the glucocorticoid component of the antiviral/immunomodulatory combination whose co-formulation is described in WO00/29027 results in a topical formulation with unexpected efficacy, in particular a remarkable efficacy as regards aborted lesions.

Accordingly, a first aspect of the invention provides a glucocorticoid-free topical composition comprising 1 to 12 weight percent of at least one acyclic guanosine analogue selected from acyclovir, penciclovir and omaciclovir in an oil-in-water or water-in-oil pharmaceutical carrier comprising, relative to the total weight of the composition, 15 to 25 weight per cent propylene glycol and 10 to 25 weight per cent isopropyl C₁₂-C₂₂ alkanoic acid ester, wherein the composition is substantially free of TCH.

The compositions of the invention are useful for the treatment or prophylaxis of diseases caused by members of the herpesvirus family, such as herpes simplex type 1 (predominantly an orofacial infection), herpes simplex type 2 (predominantly a genitoanal infection), varicella zoster virus primary infection (chicken pox) and secondary infection (shingles), human herpesvirus type 6 and 8 (implicated in the skin condition Kaposi's sarcoma) and the like. Prophylaxis in the context of the invention includes prevention of infection (including preventing spread to adjacent healthy tissue) and preventing reactivation of previous herpes virus infection, such as reactivation of herpes lying dormant in neural tissue.

As described in Biological Example 1 below, a large scale phase III clinical trial was carried out in North America during 2007 and demonstrated that treatment with the composition of the invention, commenced at the first sign of a herpes reoccurrence, results in a high proportion of patients failing to develop a cold sore lesion - i.e. an aborted lesion. No other glucocorticoid-free treatment regime has shown this beneficial effect.

Compositions of the invention may, for example, be expected to provide one or more of the following clinical benefits: a reduction in episode duration, a reduction the in pain associated with an episode, reduction in lesion severity (e.g. maximum lesion size) or the prevention of lesion development (i.e. aborted lesions).

A further aspect of the invention thus provides the use of the composition defined above in medicine, particularly in the manufacture of a topical medicament for the treatment or prophylaxis of herpes virus infections in humans, especially herpes simplex type 1 and herpes simplex type 2.

Additionally provided is a composition of the invention for use in the treatment or prophylaxis of herpes virus infections in humans (e.g. herpes simplex type 1 or, alternatively, herpes simplex type 2).

Conveniently, treatment with the composition of the invention is commenced as soon as the first sign of a herpes reoccurrence is detected, such as a tingling of the oral lesion or other manifestation of the prodromal stage. Advantageously the treatment results in an aborted lesion.

Weight percentages herein refer to the weight of the component relative to the total weight of the composition.

The expression "glucocorticoid-free" as used herein means that the pharmaceutical composition is substantially devoid of glucocorticoids, including hydrocortisone and its esters, clobetasone, triamcinolone acetonide, betmethasone, budenoside, desoximethasone, diflorosane, fluocinolone, fluoccinonide acetonide, fluocortolone, fluticasone, methylprednisolone aceponate, mometasone, rofleponide and the like. Preferably the pharmaceutical composition comprises less than 0.1 weight percent, such as <0.01% (for example less than 0.001 %) of such contaminants. If present, such contaminants will be present in an amount which is not of therapeutic significance, although most suitably the compositions of the invention will be absent of such contaminants.

The antiviral agent is included in the formulation in substantially conventional concentrations for the respective nucleoside, for example 2 to 10 weight percent, preferably 4 to 7 weight percent such as 4 or 5 weight per cent. Advantageously the formulation is largely or completely saturated with respect to the antiviral agent.

The antiviral component of the composition of the invention may comprise a mixture of acyclovir, penciclovir and/or omaciclovir, but is preferably pure acyclovir, pure penciclovir or pure omaciclovir. The low molecular weight, low toxicity and inexpensiveness of acyclovir is preferred for some embodiments. The antiviral potency of penciclovir is preferred for certain other embodiments. Omaciclovir is particularly useful where shingles lesions are suspected.

The antiviral component (s) may be in substantially dissolved form, dependent upon the carrier, but are conveniently prepared from a micronised raw material, such as those having > 75 %, preferably greater than 90% of particles with less than a defined particle size. The antiviral acyclovir or penciclovir is conveniently presented with a particle size less than 15 µm, preferably less than 7 µm.

The compositions of the invention are substantially free of 2-acetylpyridine-5-[(2-chloroanilino)thiocarbonyl]thiocarbonohydrazone (also known as BW348U87 or TCH). The expression substantially free of TCH" as used herein means that TCH will not be present in an amount which is of therapeutic significance. Suitably, the pharmaceutical composition comprises less than 0.1 weight percent, such as less than 0.01% (for example less than 0.001 %) TCH. Most suitably, the compositions of the invention will be absent of TCH.

In certain embodiments of the invention, the compositions are substantially free of pharmaceutical agents other than acyclovir, penciclovir and/or omaciclovir. The expression "substantially free of pharmaceutical agents other than acyclovir, penciclovir and/or omaciclovir" as used herein means that the specified acyclic guanosine analogues (i.e. acyclovir, penciclovir and/or omaciclovir) will be the only pharmaceutical agents present in amounts which are of therapeutic significance. Suitably, the pharmaceutical composition comprises less than 0.1 weight percent, such as less than 0.01 % (for example less than 0.001 %) of pharmaceutical agents other than the specified acyclic guanosine analogues. Most suitably, the compositions of the invention will be absent of pharmaceutical agents other than acyclovir, penciclovir and/or omaciclovir (e.g. in one embodiment of the invention acyclovir is the sole pharmaceutical agent present in the composition, in a second embodiment of the invention penciclovir is the sole pharmaceutical agent present in the composition), in a third embodiment of the invention omaciclovir is the sole pharmaceutical agent present in the composition).

In general the compositions of the invention are biphasic and comprise discrete oil and aqueous phases, either as an oil in water or a water in oil emulsion. Preferably the composition comprises a dispersed oil phase and a continuous aqueous phase. The isopropyl alkanoic acid ester will be preferentially be found in the oil phase, while the antiviral nucleoside will generally be found in the aqueous phase, typically in conjunction with the propylene glycol.

Components of the oil phase may include conventional fats and oils and their esters, as found in the European and other pharmacopeaias. Oil phase components are preferably non-greasy, non staining and washable. Conventional pharmaceutical oil phase components include mineral oils such as vaseline, liquid paraffin and the like, alkanoic acids such as stearic acid and fatty alcohols such as cetostearyl alcohol, straight or branched chain mono or dibasic alkyl esters such as di-isopropyl adipate, isocetyl stearate, propylene glycol diester of coconut fatty acids, decyl oleate, butyl stearate, 2-ethylhexyl palmitate and other 2-ethylhexanoic acid esters and the like.

Preferred isopropyl alkanoic acid esters include the dodecanate, myristate, palmitate, stearate, eicosanate or behenoate esters (and combinations thereof), in particular dodecanate, myristate and palmitate esters (and combinations thereof), especially isopropyl myristate. The composition of the invention comprises 10 to 25 weight per cent of the isopropyl alkanoic acid ester, preferably 12 to 18 weight per cent, such as 15 weight per cent.

The composition of the invention comprises 15 to 25 weight percent propylene glycol, such as 18 to 22 weight per cent. Conveniently the propylene glycol content is 20 weight per cent as this concentration generally assures a good preservative effect without needing exogenous preservatives in the composition.

The composition of the invention conveniently includes an emulsifier (surfactant), typically in an amount of 0.05 to 5, preferably 0.1 to 1 weight per cent. The European Pharmacopeia describes a number of pharmaceutically acceptable emulsifiers including anionic, cationic and non-ionic emulsifiers.

Exemplary non-ionic emulsifiers include cetomacrogols, such as cetomacrogol 1000, ethylene or diethylene glycol monostearate, glyceryl esters such as the behenate, oleate, stearate etc, laureth compounds such as lauromacrogols, macrogol monomethyl ethers, mono- and diglycerides, nonoxinols, octoxinols, poloxamers such as poloxamer 407, polyoxyl castor oils, polyoxyl stearates, polysorbates, polyvinyl alcohols, propylene glycol diacetates, sorbitan esters and the like. Poloxamer 188 is a preferred non-ionic surfactant.

Exemplary anionic emulsifiers include aluminium monostearate, calcium stearate, sulphated castor oil, magnesium stearate, pendecamaine, sodium oleate, sodium stearate, sodium stearyl fumarate, sodium tetradecyl sulphate, zinc stearate and the like. A preferred anionic emulsifier is sodium lauryl sulphate

Advantageously, the composition of the invention is buffered, for example to a pH in the range 4 to 7.5, preferably 5. Typically the buffer or buffer system is present in an amount of 0.02 - 2 weight percent, such as 0.1 to 0.2 weight percent, for example 0.14 or 0.15 weight percent. The European, US and British Pharmacopiea describe many appropriate pharmaceutically acceptable buffer systems including phosphates or ammonium acetate. A citric acid buffer, for example citric acid monohydrate, is convenient, typically in conjunction with a base in the range 0.1 to 1 weight %, such as NaOH, for example 0.06 weight %.

The compositions of the invention can also include conventional auxiliaries such as surface anaesthetics, sunscreens, flavours, scents, emollients or skin tone colourants and masks.

The compositions of the invention can be prepared by conventional blending techniques. Preferably the compositions are prepared by conventional biphasic blending techniques, whereby the oil and aqueous/propylene glycol phases are separately blended and homogenised and brought to a common temperature before mixing. The active ingredients (that is the nucleoside analogue and any additional non-glucocorticoid active) may be added to their respective oil and aqueous phases before or after blending. Preferably, to minimize the tendency to recrystallisation, the active ingredients are added after blending of the two phases. This means that there is a greater volume when the active ingredients are added, and additionally the biphasic mixture is generally at a lower temperature.

A further aspect of the invention thus provides a method for the preparation of an antiviral composition comprising bringing an oil phase comprising 10-25 weight percent (relative to the total weight of the intended formulation) isopropyl alkanoic acid ester to a defined temperature, bringing an aqueous phase comprising 15-25 weight per cent (relative to the total weight of the intended formulation) propylene glycol to the defined temperature, blending and optionally homogenising the two phases, optionally allowing the blend to cool to a lower temperature, adding effective amounts of an a guanosine nucleoside analogue antiviral agent and homogenising the resultant blend.

The intended viral conditions, such as herpes simplex lesions on the lips and/or genitalia or herpes zoster (shingles), are episodic. As with all antiviral treatments it is desirable to commence application of the medicament as soon as possible after the reactivation of a dormant herpes infection into an incipient lesion is sensed or suspected, that is the prodromal stage. For instance many people experience a warmth or tingling at the coming focal point one or more days before the first visual signs of a herpes lesion become discernable. Application of the composition of the invention is preferably commenced at this point. In some patients, exposure to certain stimuli, such as UV light when skiing or from tropical sun, severe emotional stress or menstruation, can induce reactivation of herpes lesions in particular positions. The composition of the invention can be applied in a prophylactic manner upon exposure to these stimuli. In either event it will be convenient for people prone to herpes lesions to keep a supply of the composition readily available for speedy application when needed. Accordingly it is desirable for the composition of the invention to have a long shelf life without refrigeration, so that the medicament can be kept at home or at work and/or packed for travel.

The composition will generally be applied to the incipient or apparent lesion two to twelve times per day during an episode, such as every three hours. Application preferably continues at least until the hard scab stage (if any) which generally takes 3 to 10 days from the first sensation that an episode is expected.

The composition of the invention is preferably presented in a tube containing 0.25 to 50 ml. Conveniently the tube contains sufficient for a single cold or genital sore episode, such as 1 to 5 ml. This will allow several daily applications over no more than a week or ten days, the residue suitably being discarded, thus minimizing potential contamination of the open tube and/or cross infection between individuals sharing the same tube.

A composition of particular interest consists essentially of the following ingredients:

| | | | |
|---|---|---|---|
| oil phase | | | |
| | cetostearyl alcohol | 6.75 g | 6.75% |
| | white petrolatum | 10.00 g | 10.0% |
| | liquid paraffin | 5.65 g | 5.65% |
| | isopropyl myristate | 15.00 g | 15.0% |
| aqueous phase | | | |
| | propylene glycol | 20.00 g | 20.0% |
| | sodium lauryl sulphate | 0.80 g | 0.8% |
| | poloxamer 188 | 1.00 g | 1.0% |
| | citric acid monohydrate | 0.14 g | 0.14% |
| | sodium hydroxide | 0.06 g | 0.06% |
| | | & q.s. for pH adjustment | |
| | hydrochloric acid | q.s. for pH adjustment | |
| | water, purified | q.s to 100 | |
| active component | | | |
| | acyclovir | 5.00 g | 5.0% |

at a pH suitable for topical administration (e.g. pH 5.0).

A further composition of particular interest consists essentially of the following ingredients:

| | | | |
|---|---|---|---|
| oil phase | | | |
| | cetostearyl alcohol | 6.75 g | 6.75% |
| | vaseline | 10.00 g | 10.0% |
| | liquid paraffin | 5.65 g | 5.65% |
| | isopropyl myristate | 15.00 g | 15.0% |
| aqueous phase | | | |
| | propylene glycol | 20.00 g | 20.0% |
| | sodium lauryl sulphate | 0.80 g | 0.8% |
| | poloxamer 188 | 1.00 g | 1.0% |
| | aq. purif. | q.s. to 100 | |
| active components | | | |
| | penciclovir | 5.00 g | 5.0%. |

*In vitro* skin penetration can be monitored in skin samples mounted in a two chamber diffusion cell system (Aulton ME Ed (1988) Pharmaceutics; the Science of Dosage Form Design. Churchhill Livingstone, London). In short the backs of Dunkin Harley guinea pigs are plucked, shaved and depilated with Opilca (R) as described in Alenius & Öberg (1978) Archives of Virology 58: 277-288. Two days after depilation, full thickness skin is removed and frozen at -70°C. The subcutaneous fat is removed by blunt dissection priori to mounting in the cell. The upper chamber is generally left open to facilitate cream application, typically over a surface area of 0.93 mm². The receiving chamber will generally contain Ringer solution. Samples from various times after application of cream are analysed for antiviral migration, for example by HPLC with 254 nM UV detection, mobile phase 0.05M (NH₄)H₂PO₄ buffer at pH 7.00 & 15% methanol in a 150 x 2.1 mm C₁₈ Zorbax 5 uM particle size reverse phase column.

Preclinical efficacy of compositions of the invention can be assayed as shown in the examples or with the adoptive transfer of immunity model described in WO96/24355 and WO96/24963

### Detailed Description

### Example 1

A composition in accordance with the invention is prepared from the following ingredients:

| | | | |
|---|---|---|---|
| oil phase | | | |
| | cetostearyl alcohol | 6.75 g | 6.75% |
| | white petrolatum | 10.00 g | 10.0% |
| | liquid paraffin | 5.65 g | 5.65% |
| | isopropyl myristate | 15.00 g | 15.0% |
| aqueous phase | | | |
| | propylene glycol | 20.00 g | 20.0% |
| | sodium lauryl sulphate | 0.80 g | 0.8% |
| | poloxamer 188 | 1.00 g | 1.0% |
| | citric acid monohydrate | 0.14 g | 0.14% |
| | sodium hydroxide | 0.06 g | 0.06% |
| | | & q.s. for pH adjustment | |
| | hydrochloric acid | q.s. for pH adjustment | |
| | water, purified | q.s to 100 | |
| active component | | | |
| | acyclovir | 5.00 g | 5.0% |

The particle size of the acyclovir (Recordati micronised, USP23/BP93/Eur Ph III) was 10% = 2 µm, 50% = 4 µm, 90% = 7 µm & 100% = 15 µm. The purified water is reverse osmosis treated. The pH is adjusted to 5.

The oil phase and aqueous phase components are added to respective mixing vessels, which are each heated to 70°C under agitation. When the phases are at an identical temperature, the oil phase is poured onto the aqueous phase from above while continuing to agitate for 3-5 minutes at the highest possible speed which avoids drawing air into the mixture. The thus emulsified mixture is then homogenised and cooled, with continued agitation, to 32-25°C. The active ingredients are added and agitation continued until the active ingredients are wetted and blended in. The mixture is once again homogenised and cooled until the cream thickens, around 30°C, before packaging.

### Example 2

A penciclovir composition according to the invention is prepared from the following components:

| | | | |
|---|---|---|---|
| oil phase | | | |
| | cetostearyl alcohol | 6.75 g | 6.75% |
| | vaseline | 10.00 g | 10.0% |
| | liquid paraffin | 5.65 g | 5.65% |
| | isopropyl myristate | 15.00 g | 15.0% |
| aqueous phase | | | |
| | propylene glycol | 20.00 g | 20.0% |
| | sodium lauryl sulphate | 0.80 g | 0.8% |
| | poloxamer 188 | 1.00 g | 1.0% |
| | aq. purif. | q.s. to 100 | |
| active components | | | |
| | penciclovir | 5.00 g | 5.0% |

The purified water is reverse osmosis treated. The penciclovir is micronised to mean diameter 5 µm.

The oil phase and aqueous phase components are added to respective mixing vessels, which are each heated to 70°C under agitation. When the phases are at an identical temperature, the oil phase is poured onto the aqueous phase from above while continuing to agitate for 3-5 minutes at the highest possible speed which avoids drawing air into the mixture. The thus emulsified mixture is then homogenised and cooled, with continued agitation, to 32-25°C. The active ingredients are added and agitation continued until the active ingredients are wetted and blended in. The mixture is once again homogenised and cooled until the cream thickens, around 30°C, before packaging.

### Biological Example 1

A randomized, double-blind, vehicle controlled, subject initiated phase III study looking at efficacy and safety the composition of the invention for treatment of recurrent herpes simplex labialis was undertaken under the management of Christopher M Hull, MD of the Department of Dermatology School of Medicine, University of Utah. The study took place at over 22 sites in US and Canada during the period July 2006-December 2007. The study subjects were adult, immunocompetent male or female patients with a history of at least three episodes of recurrent labial herpes over the preceding 12 months. Inclusion criteria further included a history of at least 50% episodes associated with prodromal symptoms, and at least 75% of herpes recurrences producing ulcerative lesions (that is a recurrence leading to development of a lesion which undergoes vesicle, ulcer/soft crust and/or hard crust formation. Patients agreed to refrain from using other topical medical or OTC products around the oral area during the herpes recurrence and to avoid mechanical disruption of the area affected by herpes labiales.

Exclusion criteria included systemic or topical treatment with antivirals or immunosuppressive agents within 2 weeks of randomisation, previous vaccination against herpes simplex, bearers of acyclovir resistant HSV-1, participation in concurrent trials or history of significant skin conditions that would interfere with assessment of lesions, such as atopic dermatitis, acne, eczema, psoriasis, chronic vesiculobullous disorders or rosacea.

The test product was as described in Example 1 above, applied topically five times daily during five days. The number of patients treated was 610. The comparator was prepared analogously to Example 1, but lacked the acyclovir. The number of patients treated was 232.

The primary efficacy endpoint was the proportion of subjects with non-ulcerative recurrences measured as the proportion of subjects in whom the study recurrence does not progress beyond the papule stage.

Secondary efficacy endpoints: Episode duration measured from start of treatment to loss of hard crust for an ulcerative recurrence and from start of treatment to time of no signs or symptoms for a non-ulcerative recurrence. Episode duration to normal skin, measured from start of treatment to normal skin for an ulcerative recurrence, and from start of treatment to time of no signs or symptoms for a non-ulcerative recurrence.

Tertiary efficacy endpoints: cumulative lesion area, lesion healing time to normal skin, lesion healing time to loss of hard crust, maximum lesion area, duration and severity of tenderness, and subject preference.

The subjects were asked to initiate treatment within one hour of experiencing signs or symptoms of a herpes recurrence, i.e. at the earliest prodromal symptoms or erythema but prior to any later clinical stages of a cold sore i.e. no swelling, blister or later stage present. The subjects were asked to record lesion stage, tenderness and any concomitant medication twice daily in a subject diary (*subject-observation*). The diary was also be used to record each application of study drug. The subject should visit a study clinic as soon as possible after treatment initiation, but no later than midnight of the following day, for assessment of the lesion by an investigator. Subjects who forgot or could not initiate treatment within one hour after experiencing the first signs or symptoms of a herpes lesion recurrence, or had intra oral lesions, or who had reached the papule- or later recurrence stages before treatment initiation, or who could not visit the clinic within the specified time frame were advised not to initiate treatment and to wait until their next herpes recurrence.

Visits to the clinic continued every day during the five-day treatment period for both ulcerative and non-ulcerative recurrences. For ulcerative recurrences, daily visits are required up to and including the stage "loss of hard crust" and thereafter every other day (excluding weekends) until the stage "normal skin". For non-ulcerative recurrences, visits every other day (excluding weekends) are required until "no signs or symptoms". All subjects had a follow-up interview by telephone 3 weeks (+/- 1 week) after their herpes recurrence had healed completely ("normal skin" or "no signs or symptoms"). At each visit to the clinic, the investigator observes and assesses the presence and status of herpes recurrence (prodrome, erythema (macule), papule, vesicle, ulcer, soft crust, hard crust, loss of hard crust, residual abnormalities, or normal skin). The investigator also measures ulcerative lesion size. These assessments (*investigator-observation*) were made independently of the subject's records. The investigator subsequently reviewed and discussed the subject's observations and made a third assessment based on all available information (*investigator-assessment*). This latter assessment, investigator-assessment, which includes the subject's observations on loss of hard crust and tenderness, was entered into the database for evaluations.

Viral samples (swabs) were obtained from all subjects with ulcerative recurrences in the ulcer/soft crust stages. Swabs were not obtained from lesions in the vesicle or hard crust or later stages due to the risk of disturbing the healing process. Samples were cultured at a central laboratory, and a qualitative analysis performed. Following the analysis of the clinical data, subjects from treatment groups who have a positive virus culture obtained at a later time point than the median healing time (time to loss of hard crust) in the acyclovir control group can be assessed for acyclovir susceptibility according to the standard US antiviral susceptibility testing procedure for herpes simplex virus and the genotypic nature characterized.

The schedule of events is shown in the table below. Following a screening evaluation and dispensing of study medication, subjects initiate treatment themselves within one hour of the first signs of a herpes recurrence, and visit the clinic as soon as possible after treatment initiation, but no later than midnight of the following day, as described in the methodology section.

### Schedule of events: screening, treatment, observation and follow-up periods:

| | **Screening visit** | **Treatment period,** five days for **all subjects** | **Observation period** (for **ulcerative** recurrences ) | **Observation period** (for **non-ulcerative** recurrences ) | **Follow-up period** |
|---|---|---|---|---|---|
| | | Visits every day | Visits every day until "loss of hard crust", thereafter every other day⁴ until "normal skin" | Visits every other day⁴ until "no signs or symptoms". | Follow-up by phone 3 weeks (+/- 1 week) after healing to normal skin/no signs or symptoms. |
| **Informed consent** | X | | | | |
| **Eligibility criteria¹** | X | | | | |
| **Symptoms-driven Physical examination** | X (if applicable) | | | | |
| **Medical & herpes hist.** | X | | | | |
| **Demographics recorded** | X | | | | |
| **Pregnancy test** | X | X (first visit) | | | |
| **Randomization** | X | | | | |
| **Study diary instr.** | X | | | | |
| **Dispense of study drug** | X | | | | |
| **Admin. of study drug** | | X | | | |
| **Recurrence stage assess.** | | X | X | X | |
| **Lesion size assessment** | | X | X | | |
| **Tenderness assessment** | | X | X | X | |
| **Concomitant medication** | | X | X | X | X |
| **Use & check of diary** | | X | X | X | |
| **Adverse events** | | X | X | X | X |
| **Viral isolation²** | | X | X | | |
| **Drug accountability**³ | | | X | X | |

| | | | | | |
|---|---|---|---|---|---|
| ¹Eligibility criteria will also be checked at monthly contacts with subjects. ²Only for ulcerative recurrences. Viral swabbing of crusted lesions will not be performed. ³First day of observation period. ⁴Excluding weekends | | | | | |

### Specification of recordings during the treatment and observation periods:

| | **Subject observation** (recorded in subject diary) | **Investigator observation** (recorded in CRF) | **Investigator assessed** (recorded in CRF) |
|---|---|---|---|
| **Study drug administration** | X | | X |
| **Assessment of recurrence stage** | X | X | X |
| **Lesion size assessment** | | X | X |
| **Tenderness assessment** | X | X | X |
| **Concomitant medication** | | | X |
| **Check of diary** | | | X |
| **Adverse events** | | X | X |
| **Viral isolation** | | | X |
| **Drug accountability** | | | X |

The primary efficacy endpoint - prevention in the ITT (intention to treat) population was 35.4% in the arm of the study treated with the composition of the invention, with a P value relative to the control of 0.011. This means that treatment with the invention led to over one third of patients not developing a herpes lesion at all. Those patients which did develop a lesion (secondary and tertiary endpoints above) also had satisfactory reductions of the lesion duration, size and/or pain relative to control, for example an average of 0.7 day reduction in episode duration. The remarkable result as regards aborted lesions should be compared with the large scale phase III clinical trials described in the Spruance reference (2002 Antimicrob. Agents Chemother. 46(7) 2238-2243) referred to above, where the most widely marketed acyclovir cream, i.e. 5% acyclovir in the 40% propylene glycol MAC vehicle, did not prevent the development of classical lesions.

## Claims

1. A glucocorticoid-free topical antiviral composition comprising 1 to 12 weight percent of at least one acyclic guanosine analogue selected from acyclovir, penciclovir and omaciclovir in an oil-in-water or water-in-oil pharmaceutical carrier comprising 15 to 25 weight per cent propylene glycol and 10 to 25 weight per cent isopropyl C₁₂-C₂₂ alkanoic acid ester, wherein each reference to weight percent is relative to the entire weight of the composition, wherein the composition is substantially free of TCH.

2. A composition according to claim 1, wherein the composition is substantially free of pharmaceutical agents other than acyclovir, penciclovir and/or omaciclovir.

3. A composition according to either of claims 1 or 2, wherein the acyclic guanosine analogue is acyclovir.

4. A composition according to claim 3, wherein acyclovir is the sole pharmaceutical agent present.

5. A composition according to claim 2, wherein penciclovir is the sole pharmaceutical agent present.

6. A composition according to any one of claims 1 to 5, wherein the carrier comprises 18 to 22 weight per cent propylene glycol, preferably 20 weight per cent.

7. A composition according to any one of claims 1 to 6, wherein the carrier comprises 12 to 18 weight per cent isopropyl alkanoic acid ester, preferably 15 weight per cent.

8. A composition according to claim 7, wherein the isopropyl alkanoic acid ester is selected from the group dodecanate, myristate, palmitate, stearate, eicosanate or behenoate esters, or mixtures thereof.

9. A composition according to claim 8 wherein the isopropyl alkanoic ester is isopropyl myristate.

10. A composition according to any one of claims 1 to 9, wherein the guanosine nucleoside analogue comprises 4-7 weight percent, preferably 5 weight percent.

11. A composition according to any one of claims 1 to 10, in the form of an oil-in-water emulsion.

12. A composition according to any one of claims 1 to 11 for use in the treatment or prophylaxis of herpes virus infection.

13. A composition according to claim 12 for use in reduction of episode duration.

14. A composition according to claim 12 for use in reduction of pain associated with an episode.

15. A composition according to claim 12 for use in reduction of lesion severity.

16. A composition according to claim 12 for use in prevention of lesion development.

17. A composition according to claim 12, for use wherein administration occurs at the prodromal stage of a herpes reoccurence.

18. Use of a composition according to any one of claims 1 to 11 in the manufacture of a topical medicament for the treatment or prophylaxis of herpes virus infections in humans.

## Patentansprüche

1. Glucocorticoid-freie topische antivirale Zusammensetzung, umfassend 1 bis 12 Gewichtsprozent wenigstens eines acyclischen Guanosinanalogs, ausgewählt aus Acyclovir, Penciclovir und Omaciclovir, in einem pharmazeutischen Öl-in-Wasser- oder Wasser-in-ÖI-Trägermittel, enthaltend 15 bis 25 Gewichtsprozent Propylenglykol und 10 bis 25 Gewichtsprozent C₁₂-C₂₂-Alkansäureisopropylester, wobei sich die Gewichtsprozentangaben jeweils auf das Gesamtgewicht der Zusammensetzung beziehen, wobei die Zusammensetzung weitgehend frei von TCH ist.

2. Zusammensetzung nach Anspruch 1, wobei die Zusammensetzung weitgehend frei von anderen Pharmazeutika als Acyclovir, Penciclovir und/oder Omaciclovir ist.

3. Zusammensetzung nach Anspruch 1 oder 2, wobei es sich bei dem acyclischen Guanosinanalog um Acyclovir handelt.

4. Zusammensetzung nach Anspruch 3, wobei Acyclovir als einziges Pharmazeutikum vorliegt.

5. Zusammensetzung nach Anspruch 2, wobei Penciclovir als einziges Pharmazeutikum vorliegt.

6. Zusammensetzung nach einem der Ansprüche 1 bis 5, wobei das Trägermittel 18 bis 22 Gewichtsprozent Propylenglykol, vorzugsweise 20 Gewichtsprozent, enthält.

7. Zusammensetzung nach einem der Ansprüche 1 bis 6, wobei das Trägermittel 12 bis 18 Gewichtsprozent Alkansäureisopropylester, vorzugsweise 15 Gewichtsprozent, enthält.

8. Zusammensetzung nach Anspruch 7, wobei der Alkansäureisopropylester ausgewählt ist aus der Gruppe Dodekan-, Myristin-, Palmitin-, Stearin-, Eicosan- oder Behensäureester oder Gemischen davon.

9. Zusammensetzung nach Anspruch 8, wobei es sich bei dem Alkansäureisopropylester um Myristinsäureisopropylester handelt.

10. Zusammensetzung nach einem der Ansprüche 1 bis 9, wobei das Guanosin-Nukleosidanalog 4-7 Gewichtsprozent, vorzugsweise 5 Gewichtsprozent, umfasst.

11. Zusammensetzung nach einem der Ansprüche 1 bis 10, in Form einer Öl-in-Wasser-Emulsion.

12. Zusammensetzung nach einem der Ansprüche 1 bis 11 zur Verwendung bei der Behandlung oder Vorbeugung einer Herpesvirusinfektion.

13. Zusammensetzung nach Anspruch 12 zur Verwendung bei der Verringerung der Episodendauer.

14. Zusammensetzung nach Anspruch 12 zur Verwendung bei der Verringerung von Schmerzen in Zusammenhang mit einer Episode.

15. Zusammensetzung nach Anspruch 12 zur Verwendung bei der Verringerung der Schwere von Läsionen.

16. Zusammensetzung nach Anspruch 12 zur Verwendung bei der Verhinderung der Entwicklung von Läsionen.

17. Zusammensetzung nach Anspruch 12 zur Verwendung, wobei die Verabreichung im Prodromalstadium eines Wiederauftretens von Herpes erfolgt.

18. Verwendung einer Zusammensetzung nach einem der Ansprüche 1 bis 11 bei der Herstellung eines topischen Arzneimittels zur Behandlung oder Vorbeugung von Herpesvirusinfektionen beim Menschen.

## Revendications

1. Composition antivirale topique sans glucocorticoïde comprenant de 1 à 12 pour cent en poids d'au moins un analogue de guanosine acyclique choisi parmi l'acyclovir, le penciclovir et l'omaciclovir dans un véhicule pharmaceutique d'huile dans l'eau ou d'eau dans l'huile comprenant de 15 à 25 pour cent en poids de propylèneglycol et de 10 à 25 pour cent en poids d'ester d'acide alcanoïque en C₁₂-C₂₂ isopropylique, où chaque référence à un pourcentage en poids est relatif au poids total de la composition, où la composition est sensiblement exempte de TCH.

2. Composition selon la revendication 1, la composition étant sensiblement exempte d'agents pharmaceutiques autres que l'acyclovir, le penciclovir et/ou l'omaciclovir.

3. Composition selon l'une quelconque des revendications 1 ou 2, où l'analogue de guanosine acyclique est l'acyclovir.

4. Composition selon la revendication 3, où l'acyclovir est le seul agent pharmaceutique présent.

5. Composition selon la revendication 2, où le penciclovir est le seul agent pharmaceutique présent.

6. Composition selon l'une quelconque des revendications 1 à 5, où le véhicule comprend de 18 à 22 pour cent en poids de propylèneglycol, de préférence 20 pour cent en poids.

7. Composition selon l'une quelconque des revendications 1 à 6, où le véhicule comprend de 12 à 18 pour cent en poids d'ester d'acide alcanoïque isopropylique, de préférence 15 pour cent en poids.

8. Composition selon la revendication 7, où l'ester d'acide alcanoïque isopropylique est choisi dans le groupe des esters dodécanate, myristate, palmitate, stéarate, eicosanate ou béhénoate, ou des mélanges de ceux-ci.

9. Composition selon la revendication 8 où l'ester d'acide alcanoïque isopropylique est le myristate d'isopropyle.

10. Composition selon l'une quelconque des revendications 1 à 9, où l'analogue de guanosine acyclique comprend de 4 à 7 pour cent en poids, de préférence 5 pour cent en poids.

11. Composition selon l'une quelconque des revendications 1 à 10, sous la forme d'une émulsion d'huile dans l'eau.

12. Composition selon l'une quelconque des revendications 1 à 11 pour utilisation dans le traitement ou la prophylaxie d'une infection par le virus de l'herpès.

13. Composition selon la revendication 12 pour utilisation dans la réduction de la durée des épisodes.

14. Composition selon la revendication 12 pour utilisation dans la réduction de la douleur associée à un épisode.

15. Composition selon la revendication 12 pour utilisation dans la réduction de la gravité des lésions.

16. Composition selon la revendication 12 pour utilisation dans la prévention du développement des lésions.

17. Composition selon la revendication 12, pour utilisation lorsque l'administration se produit au stade prodromique d'une récurrence d'herpès.

18. Utilisation d'une composition selon l'une quelconque des revendications 1 à 11 dans la fabrication d'un médicament topique pour le traitement ou la prophylaxie d'infections par le virus de l'herpès chez des humains.
